# EUROPEAN PATENT APPLICATION

(11) **EP 1 652 532 A1**
(43) Date of publication of application: **03.05.2006**
(21) Application number: 04747885.4
(22) Date of filing: 16.07.2004
(51) Int. Cl.: A61K 45/00, A61K 31/137, A61K 31/216, A61K 31/38, A61P 13/10, C07D 495/04

(54) **MEDICINAL COMPOSITION**

(30) Priority: 16.07.2003 JP 2003197662
(71) Applicant: KYOWA HAKKO KOGYO CO., LTD., Chiyoda-ku, Tokyo 100-8185 (JP)
(72) Inventor: YAMAGATA, T. c/o Pharmaceutical Research Center, Nagaizumi-cho Sunto-Gun Shizuoka411-8731 (JP); SHIRAKURA, S. c/o Pharmaceutical Research Center, Nagaizumi-cho Sunto-Gun Shizuoka411-8731 (JP)
(74) Representative: Vossius & Partner
(86) International application number: PCT/JP2004/010521
(87) International publication number: WO 2005/007191

(57) **Abstract**

The present invention provides a pharmaceutical composition which is useful in the treatment for overactive bladder and the like, and comprises 3,3,3-trifluoro-2-hydroxy-2-methyl-N-(5,5,10-trioxo-4,10-dihydrothieno[3,2-c][1]benzothiepin-9-yl)propanamide or a pharmaceutically acceptable salt thereof, and an anticholinergic agent.

## Description

### Technical Field

The present invention relates to a pharmaceutical composition which comprises 3,3,3-trifluoro-2-hydroxy-2-methyl-N-(5,5,10-trioxo-4,10-dihydrothieno[3,2-c][1]benzothiepin-9-yl)propanamide (hereinafter referred to as Compound (I), if desired) or a pharmaceutically acceptable salt thereof, and an anticholinergic agent.

### Background Art

Overactive bladder is a pathological condition observed inpatients referring symptoms such as urinary urgency or urinary frequency with or without urinary urge incontinence. "Urinary urgency" refers to a sudden and strong desire to void, and "urinary urge incontinence" refers to involuntary urinary leakage associated with urinary urgency.

In patients suffering from the symptoms such as urinary urgency and urinary urge incontinence associated with overactive bladder, involuntary (uninhibited) contraction of the detrusor muscle, a detrusor overactivity, is frequently observed in a cystometrogram. The detrusor overactivity is considered to be a main cause of urinary urgency and also of urinary urge incontinence, and urinary urgency can lead to urinary frequency.

Micturition reflex is physiologically controlled by the complex reflex pathways including peripheral and central nervous systems [Urology, Vol. 50, Supplement No. 6A, pp. 36-52 (1997)]. Acetylcholine is released from the pelvic nervous terminals of a parasympathetic nervous system that controls a bladder, and this binds to a receptor in detrusor layer, then urinary bladder is contracted to induce urination. The receptor of a neurotransmitter acetylcholine is referred to as a muscarine receptor, and the muscarine receptor is divided into three groups of M₁-, M₂- and M₃-receptors. It is said that M₃- or M₂-receptor is abundant in urinary bladders, and a non-selectivemuscarinic receptor antagonist or an M₃-selective muscarinic antagonist is used for therapy of pollakiuria and incontinence that are the symptoms of overactive bladder. In general, the muscarinic antagonists are referred to as an anticholinergic agent.

An anticholinergic agent is not always sufficiently satisfactory for overactive bladder patients because of its adverse effects such as dry mouth, increase in residual urine, urinary retention, diarrhea, constipation. In addition, owing to these adverse effects, a sufficient dose of anticholinergic agents could not be administered to some cases of overactive bladder.

On the other hand, various types of potassium channels exist in sensory neurons and detrusors of urinary bladder, and they modulate neuronal excitation and detrusor contraction *[The Journal ofPhysiology,* Vol. 494, No. 1, pp. 1-16 (1996) ; *Current Drug Targets,* Vol. 2, No. 1, pp. 1-20 (2001); *Acta Physiologica Scandinavica,* Vol. 173, No. 3, pp. 323-333 (2001)]. Overactive bladder is associated with excitation of sensory neurons and excitation and contraction of detrusor, and these also participate in urinary urgency and detrusor overactivity [Urology, Vol. 30, No. 5, Supplement 1, pp. 22-26 (2002)].

Heretofore, it is known that Compound (I) or a pharmaceutically acceptable salt thereof is effective for treatment of urinary incontinence (WO98/46587), and it is known that Compound (I) or a pharmaceutically acceptable salt thereof has an opening effect of A-type potassium channels and is effective for treatment of overactive bladder (WO02/078523 and WO02/078710).

### Disclosure of the Invention

An object of the present invention is to provide a pharmaceutical composition which comprises Compound (I) or a pharmaceutically acceptable salt thereof and an anticholinergic agent, or the like.

The present invention relates to the following (1) to (21) :
(1) A pharmaceutical composition which comprises (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 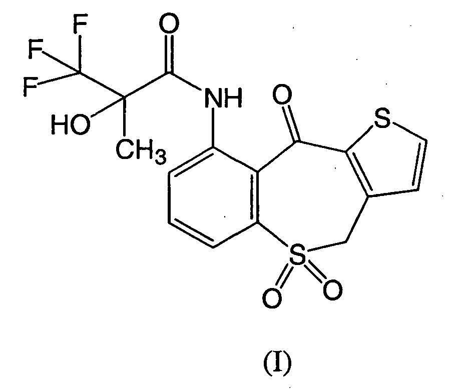 or a pharmaceutically acceptable salt thereof, and (b) an anticholinergic agent.
(2) The pharmaceutical composition according to (1), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 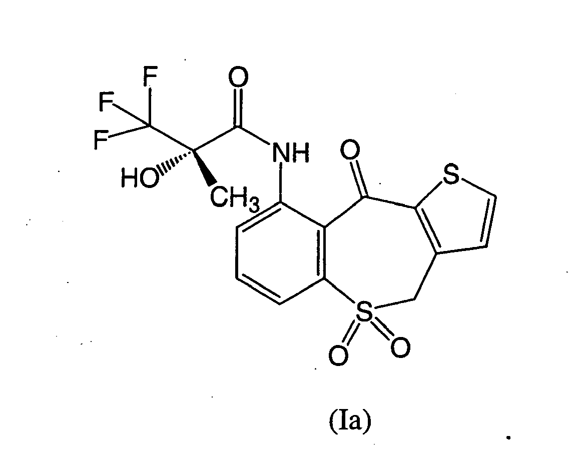
(3) The pharmaceutical composition according to (1) or (2), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(4) A therapeutic agent for overactive bladder, which comprises, as active ingredients, (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 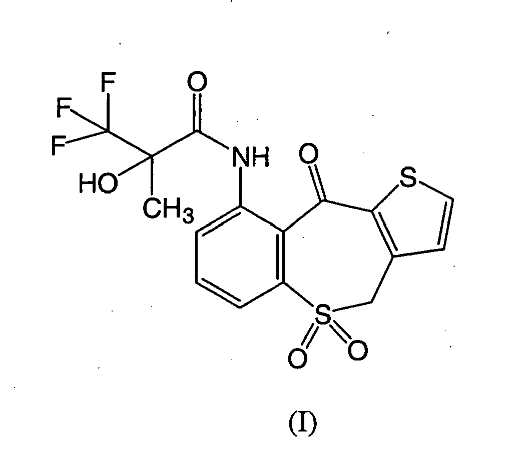 or a pharmaceutically acceptable salt thereof, and (b) an anticholinergic agent, which may be administered together or separately at an interval.
(5) The therapeutic agent for overactive bladder according to (4), wherein 3,3,3-trifluoro-2-hydroxy-2-methyl-N-(5,5,10-trioxo-4,10-dihydrothierio[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by formula (Ia): 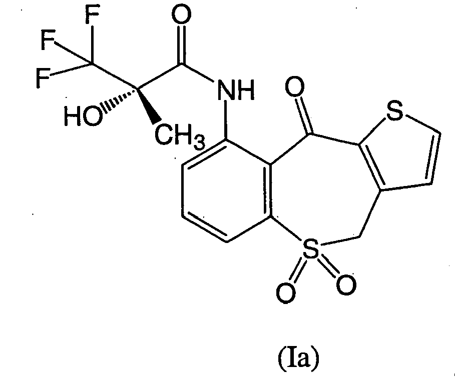
(6) The therapeutic agent for overactive bladder according to (4) or (5), wherein the anticholinergic agent is /are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(7) A kit which comprises (a) a first component containing 3,3,3-trifluoro-2-hydroxy-2-methyl-N-(5,5,10-trioxo-4,10-dihydrothieno[3,2-c][1]benzothiepin-9-yl)propanamide represented by Formula (I): 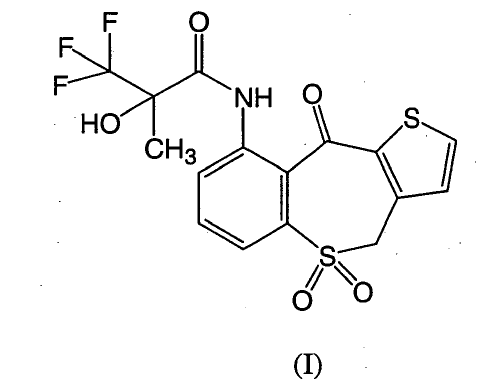 or a pharmaceutically acceptable salt thereof, and (b) a second component containing an anticholinergic agent.
(8) The kit according to (7), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-o-4,10-dihydrothieno[3,2-c][1]benzothiepin-9-yl)propanamide represented by Formula (Ia): 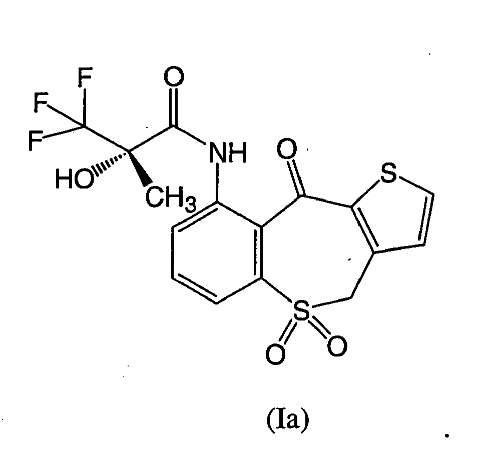
(9) The kit according to (7) or (8), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(10) A kit for treating overactive bladder, which comprises (a) a first component containing 3,3,3-trifluoro-2-hydroxy-2-methyl-N-(5,5,10-trioxo-4,10-dihydrothieno[3,2-c][1]benzothiepin-9-yl)propanamide represented by Formula (I): 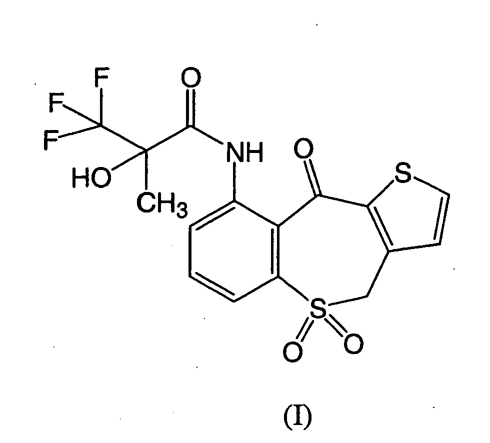 or a pharmaceutically acceptable salt thereof, and (b) a second component containing an anticholinergic agent.
(11) The kit for treating overactive bladder according to (10), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 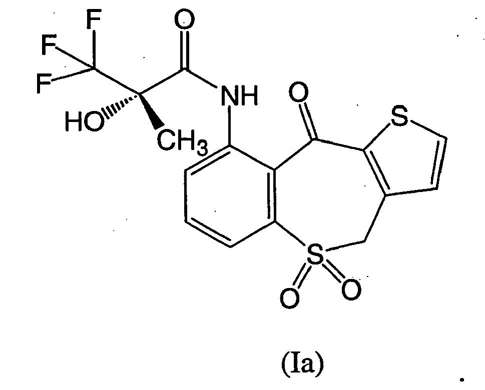
(12) The kit for treating overactive bladder according to (10) or (11), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(13) 3,3,3-Trifluoro-2-hydroxy-2-methyl-N-(5,5,10-trioxo-4,10-dihydrothieno[3,2-c][1]benzothiepin-9-yl)propanamide represented by Formula (I): 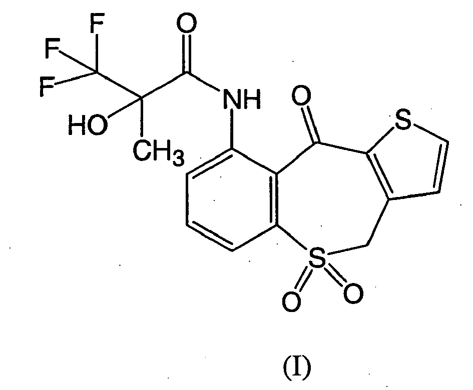 or a pharmaceutically acceptable salt thereof, which may be administered together or separately at an interval with an anticholinergic agent.
(14) 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to (13), wherein
   3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamid e represented by Formula (Ia): 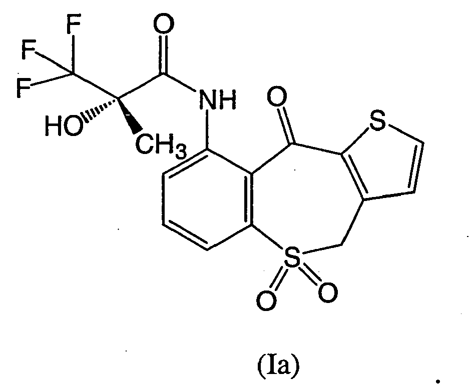
(15) 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to (13) or (14), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(16) A pharmaceutical composition which comprises, as an active ingredient,
   3,3,3-trifluoro-2-hydroxy-2-methyl-N-(5,5,10-trioxo-4,10-dihydrotlzieno[3,2-c][1]benzothiepin-9-yl)propanamide represented by Formula (I): 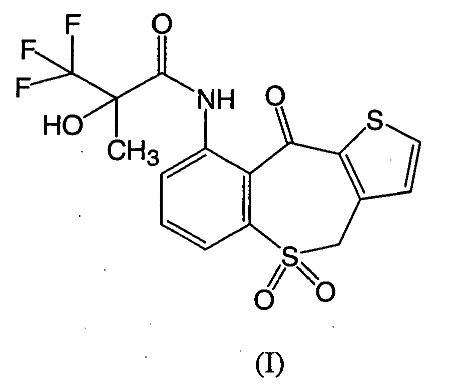 or a pharmaceutically acceptable salt thereof, which may be administered together or separately at an interval with an anticholinergic agent.
(17) The pharmaceutical composition according to (16), wherein
   3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 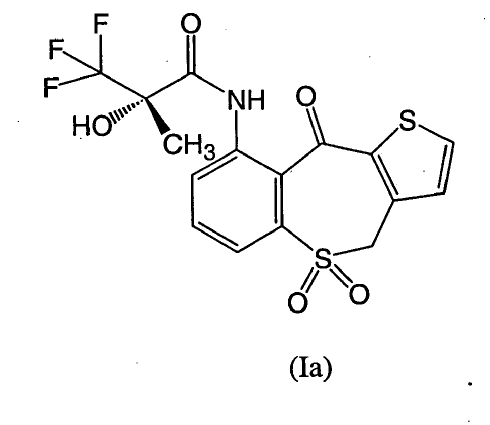
(18) The pharmaceutical composition according to (16) or (17), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
(19) A method for treating overactive bladder, which comprises administering (a)
   3,3,3-trifluoro-2-hydroxy-2-methyl-N-(5,5,10-trioxo-4,10-dihydrothieno[3,2-c][1]benzothiepin-9-yl)propanamide represented by Formula (I): 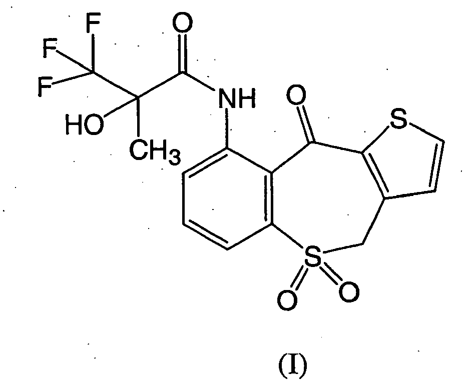 or a pharmaceutically acceptable salt thereof, and (b) an anticholinergic agent, which may be administered together or separately at an interval.
(20) The method for treating overactive bladder according to (19), wherein
   3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 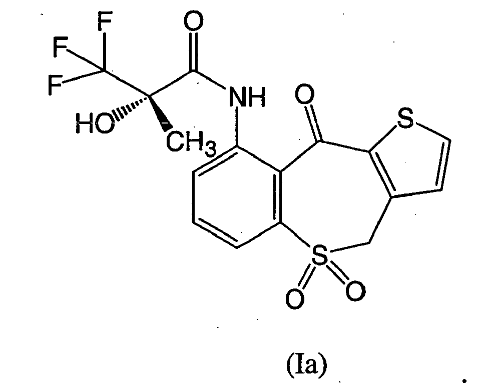
(21) The method for treating overactive bladder according to (19) or (20), wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

The pharmaceutically acceptable salt of Compound (I) includes, for example, pharmaceutically acceptable acid addition salts, metal salts, ammonium salts, organic amine addition salts, amino acid addition salts and the like.

The pharmaceutically acceptable acid addition salts of Compound (I) include inorganic acid salts such as hydrochlorides, sulfates, hydrobromides, nitrates and phosphates; and organic acid salts such as acetates, mesylates, succinates, maleates, fumarates, citrates and tartrates. The pharmaceutically acceptable metal salts include alkali metal salts such as sodium salts and potassium salts; alkaline earth metal salts such as magnesium salts and calcium salts; and aluminum salts and zinc salts. The pharmaceutically acceptable ammonium salts include ammonium salts, tetramethylammonium salts and the like. The pharmaceutically acceptable organic amine addition salts include salts with morpholine, piperidine and the like. The pharmaceutically acceptable amino acid addition salts include addition salts with glycine, phenylalanine, lysine, aspartic acid, glutamic acid and the like.

A production method of Compound (I) is described.

Compound (I) can be produced according to the method described in WO98/4658 or a similar method thereof.

There may be stereoisomers (for example, tautomers, enantiomers and the like) for Compound (I). All possible isomers and mixtures thereof including above-mentioned stereoisomers can be used as the pharmaceutical composition, the therapeutic agent for overactive bladder, the kit, the kit for treating overactive bladder and the method for treating overactive bladder of the present invention. Compound (I) of the present invention includes all possible isomers and mixtures thereof including above-mentioned stereoisomers.

To obtain a salt of Compound (I), it may be purified as it is when it is produced in the form of the salt, and when it is produced in the form of a free form, it may be dissolved or suspended in a suitable solvent, and added with an acid or a base, followed by isolation and purification.

Compound (I) and pharmaceutically acceptable salts thereof may exist in the form of adducts with water or various solvents, and such adducts can also be used as the pharmaceutical composition, the therapeutic agent for overactive bladder, the kit, the kit for treating overactive bladder and the method for treating overactive bladder of the present invention, and such adducts are included in Compound (I) and pharmaceutically acceptable salts thereof of the present invention.

The anticholinergic agents include any compounds that competitively block muscarinic receptors to inhibit the activity of cholinergic neurons. The anticholinergic agents, for example, include oxybutynin, propiverine, tolterodine, darifenacin, temiverine, trospium chloride, tiotropium, oxytropium, ipratropium, flutropium, atropine, scopolamine, solifenacin, KRP-197 [ONO-8025, Bioorganic Medicinal Chemistry, Vol.7, No.6, pp.1151-1161 (1999); Bioorganic Medicinal Chemistry Letters, Vol.8, No.14, pp.1807-1812 (1998)] and the like, as well as stereoisomers (for example, enantiomers and the like) thereof, pharmaceutically acceptable salts thereof and hydrates thereof. One or more of these may be used therein either singly or as combined. The pharmaceutically acceptable salts of the compounds are, for example, the salts mentioned hereinabove for the pharmaceutically acceptable salts of Compound (I).

Compound (I) or a pharmaceutically acceptable salt thereof and the anticholinergic agent used in the pharmaceutical composition or the therapeutic agent for overactive bladder of the present invention may be administered alone or in combination as preparations containing their active ingredients. Particularly, a combination of two or more preparations is preferable. When the preparations are used or administered in combination, they may be used or administered together or separately at an interval.

The dose ratio (weight/weight) of Compound (I) or a pharmaceutically acceptable salt thereof to the anticholinergic agent may be suitably determined, depending on the combination with the anticholinergic agent to be used and the efficacy of the anticholinergic agent. Specifically, for example, the ratio is from 1/50 (Compound (I) or pharmaceutically acceptable salt thereof/anticholinergic agent) to 50000/1, more preferably from 1/30 to 10000/1, even more preferably from 1/20 to 5000/1, still more preferably from 1/10 to 1000/1.

When the preparations are administered in combination, for example, for example, (a) a first component comprising Compound (I) or a pharmaceutically acceptable salt thereof, and (b) a second component comprising an anticholinergic agent are separately prepared as described above, and made into a kit. Byutilizingsuchakit, each component can be administered together or separately at an interval to one subject by the same route or different routes.

The kit comprises two or more containers (for example, vials, bags) and the contents. The material and the shape of the containers are not limited, but the containers must prevent the contents, i.e. the components, from degrading due to external temperature or light during the storage, and should be made from a material that does not elute its chemical constituents. The first component and the second component are administerable dosage forms so as to be administered through different routes (for example, tubes) or the same route. Specificallymentioned are a kit of tablets, injections, and the like.

The method for treating overactive bladder of the present invention may be carried out in the same manner as that for the use or administration of Compound (I) or a pharmaceutically acceptable salt thereof and an anticholinergic agent used in the pharmaceutical composition or the therapeutic agent for overactive bladder described above. That is, Compound (I) or a pharmaceutically acceptable salt thereof and an anticholinergic agent are formulated into preparations containing the respective active ingredients therein, and for example, they are administered as single preparation or as a combination of preparations, preferably, they are administered as a combination of two or more preparations. When preparat ions are administered in combination, they may be administered together or separately at an interval, and the kit described above may be used for administration.

The efficacy of treatment of overactive bladder by the combined administration of Compound (I) or a pharmaceutically acceptable salt thereof and an anticholinergic agent will be described specifically with reference to Test Example. In the following Test Example,
(S)-(+)-3,3,3-trifluoro-2-hydroxy-2-methyl-N-(5,5,10-trioxo-4,10-dihydrothieno[3,2-c][1]benzothiepin-9-yl) propanamide (hereinafter referred to as Compound (Ia), if desired) was used as Compound (I).
Test Example: Inhibitory Activity on Detrusor Overactivity

The experiment was carried out by a method similar to Cheng et al. [Brain Research, Vol. 678, pp. 40-48 (1995)].

Female SD rats of 8 to 9 weeks of age (supplied by Charles River Japan) were used in the test. Rats were housed in metal cages (5-7 animals/cage) and given commercially available chow and water *ad libitum,* kept in an animal room maintained at room temperature between 19 and 25°C and humidity between 30 and 70% under illumination for 12 hours (from 7:00 a.m. to 7:00 p.m.) per day.

Spinal cord was injured in the rats. Each animal was anesthetized with diethyl ether and the skin on the dorsal surface of thoracic cord was incised. The 7th to 8th thoracic vertebrae were laminectomised. Then, the resection around thoracic cord at the T7-T8 segments was macroscopically made by about 5 mm in length, and the wound cavity of the removed part was filled with oxidized regenerated cellulose for hemostasis. The incised part was sutured with a surgical silk. After the spinal cord injury operation, urine was expressed manually twice per day (at 8:00 - 10:00 and 17:00 - 19:00) for about 3 weeks until occurrence of autonomic micturition. An antibiotic (ampicillin, Sigma Chemical Co.) was intramuscularly administered to the animals at a dose of 150 mg/kg once per day for about 1 week.

Four to five weeks after the spinal cord injury, the rats were subj ected to intravesical implantation of catheter. Under diethyl ether anesthesia, the bladder was exposed by midline incision of the abdomen. A polyethylene tube (PE-50, Becton Dickinson), which was blunted at the tip not to damage tissue, was filled with a physiological saline (Otsuka Pharmaceutical Factory) and inserted into bladder via the apex. The intravesical catheter was fixed with a surgical silk ligature to implant. The other end of the catheter was exposed subcutaneously from the dorsal neck, plugged and then fixed to the skin with a surgical thread.

Four to eight days after the intravesical implantation of catheter, a cystometry was performed. The rats were kept in a Bollman cage (Natsume Seisakusho Co. , Ltd.) and a three-way cock was connected to the intravesical catheter, one end of the three-way cock was connected to a pressure transducer (Nihon Kohden Corp.) and the other end was connected to a 50-mL syringe (Terumo Corp.) arranged to an infusion pump (KD Scientific) for physiological saline infusion. The intravesical pressure signal from the pressure transducer was amplified with a strain pressure amplifier (AP-621G, Nihon Kohden Corp.) connected thereto, and was recorded on a thermal array recorder (RTA-1200, Nihon Kohden Corp.) via a polygraph system (RMP-6008, Nihon Kohden Corp.) containing the above amplifier. Sixty to 90 minutes after the completion of the preparation for the measurement, a room temperature physiological saline was infused into the bladder at a flow rate of 10 mL/h for 30 minutes to confirm the occurrence of micturition contractions. Thirty minutes later, the infusion of physiological saline was carried out again for 30 minutes, and the intravesical pressure was measured to be used as the pre-administration value. Compound (Ia) was suspended in a 0.5 w/v (weight/volume) % methyl cellulose solution at a concentration of 1 mg/ml. The suspension was further diluted with a 0.5 w/v % methyl cellulose solution to prepare an administration solution (Compound (Ia) administration solution) at a concentration of 0.01 mg/ml. This was orally administered to the rats at a volume of 1 ml/kg. An anticholinergic agent tolterodine was dissolved in a 0.5 w/v % methylcellulose solution at a concentration of 3 mg/ml (anticholinergic agent administration solution), and this was orally administered to the rats at a volume of 1 ml/kg each. For investigating the effect of the combination administration, the Compound (Ia) administration solution and the anticholinergic agent administration solution were simultaneously administered to the rats, both at a volume of 1 ml/kg each. The periods of 1, 3 and 5 hours after the administration were used as the measuring time after the administration of the vehicle or drug. During a duration of 15 minutes around each measuring time (45 to 75 minutes, 165 to 195 minutes, and 285 to 315 minutes after the drug administration), a physiological saline was infused into the bladder of each rat.

Premicturition contractions were measured as an index of detrusor overactivity. The average of the maximum premicturition contraction between respective micturition contractions were designated as the amplitude of premicturition contractions at each measurement point. The number of premicturition contractions for 2 minutes just before voiding was counted, and this is referred to as frequency of premicturition contractions. The amplitude and frequency of premicturition contractions were read from intravesical pressure waveforms recorded on a chart using a digitizer (KW4620, Graphtec Corporation) controlled by a computer (PC-9801NS/R, NEC), and saved as a DAT-format file or a WJ2-format file. The data files were imported into Excel 2000 (Microsoft). The amplitude and frequency of premicturition contractions were expressed as relative values when the values before the drug administration were defined as 100, and the average ± standard error was calculated for each group.

The values (%) of amplitude of premicturition contractions after vehicle (control) or drug (Compound (Ia), anticholinergic agent, and Compound (Ia) + anticholinergic agent) administration are shown in Table 1, and the values (%) of frequency of premicturition contractions are shown in Table 2.

**Table 1 : Effect of Combination of Compound (la) and Tolterodine on amplitude of premicturition contractions in rats with spinal cord injury**

| | control | Compound (la) 0.01 mg/kg, p.o. | Tolterodine 3 mg/kg, p.o. | Compound (1a) + Tolterodine |
|---|---|---|---|---|
| Before administration | 100.0 ± 0.0 | 100.0 ± 0.0 | 100.0 ± 0.0 | 100.0 ± 0.0 |
| After 1 hour | 117.5 ± 8.3 | 71.9 ± 7.3*** | 105.9 ± 24.3 | 57.3 ± 5.7*** |
| After 3 hours | 110.2 ± 10.5 | 62.6 ± 7.3** | 79.6 ± 9.7 | 37.9 ± 2.6***^{,††,‡‡} |
| After 5 hours | 90.6 ± 11.4 | 65.1 ± 7.2 | 70.3 ± 11.9* | 42.8 ± 4.1 ***^{,†} |

| | | | | |
|---|---|---|---|---|
| **P<0.01, ***P<0.001 (compared with control group) | | | | |
| ^{†}P<0.05, ^{††}P<0.01 (compared with Compound (1a) administration group) | | | | |
| ‡‡P<0.01 (compared with tolterodine administration group) (n=9; Student's t-test or Aspin-Welch test) | | | | |

**Table 2 : Effect of Combination of Compound (1a) and Tolterodine on frequency of premicturition contractions in rats with spinal cord injury**

| | control | Compound (Ia) 0.01 mg/kg, p.o. | Tolterodine 3 mg/kg, p.o. | Compound (Ia) + Tolterodine |
|---|---|---|---|---|
| Before administration | 100.0 ± 0.0 | 100.0 ± 0.0 | 100.0 ± 0.0 | 100.0 ± 0.0 |
| After 1 hour | 109.9 ± 8.0 | 61.6 ± 9.8 ** | 70.4 ± 11.6 | 48.4 ± 4.9 *** |
| After 3 hours | 90.3 ± 7.3 | 53.2 ± 4.9*** | 73.4 ± 8.2 | 39.8 ± 5.3***^{,‡‡} |
| After 5 hours | 92.2 ± 11.0 | 56.0 ± 8.3* | 75.5 ± 12.0 | 45.3 ± 4.5**^{,}‡ |

| | | | | |
|---|---|---|---|---|
| *P<0.06, **P<0.01, ***P<0.001 (compared with control group) | | | | |
| ^{‡}P<0.05, ^{‡‡}P<0.01 (compared with tolterodine administration group) (n=9; Student's t-test or Aspin-Welch test) | | | | |

Compound (Ia) and tolterodine inhibited premicturition contractions (amplitude and frequency of premicturition contractions). Combined administration of Compound (Ia) and tolterodine resulted in enhanced inhibition of premicturition contractions (amplitude and frequency of premicturition contractions).

Therefore, it is thought that a combination of Compound (I) or a pharmaceutically acceptable salt thereof and an anticholinergic agent may be useful for the treatment of overactive bladder.

As described above, the pharmaceutical composition or the therapeutic agent for overactive bladder of the present invention may be used, administered or produced in a single preparation or a combination of preparations so far as the preparations are formulated so as to contain the respective active ingredients, Compound (I) or a pharmaceutically acceptable salt thereof and an anticholinergic agent. Preferably, the pharmaceutical composition or the therapeutic agent for overactive bladder has a unit dose form suitable to oral administration such as tablets or capsule, or has a unit dose form suitable to parenteral administration such as injections. When preparations are used or administered as a combination of preparations, they may be used or administered together or separately at an interval.

These preparations may be produced in any ordinary method using any other pharmaceutically acceptable diluent, excipient, disintegrator, lubricant, binder, surfactant, water, physiological saline, vegetable oil solubilizer, isotonizing agent, preservative and antioxidant, in addition to the respective active ingredients.

In preparing tablets and capsules, for example, excipients such as lactose, disintegrators such as starch, lubricants such as magnesium stearate, binders such as hydroxypropyl cellulose, surfactants such as fatty acid ester, and plasticizers such as glycerin can be used according to any ordinary manner.

In preparing injections, for example, carriers such as distilled water, salt solution, glucose solution or a mixture of salt water and glucose solution, solubilizers, isotonizing agents, preservatives, and antioxidants can be used according to any ordinary manner.

When Compound (I) or a pharmaceutically acceptable salt thereof and an anticholinergic agent are used or administered as a combination of preparations for the obj ect described above, the dose and the administration frequency thereof may vary depending on the dosage form, the age, the body weight and the condition of patients. In general, Compound (I) or a pharmaceutically acceptable salt thereof and an anticholinergic agent are preferably administered in a dose described below per day:
Compound (I) or a pharmaceutically acceptable salt thereof can be administered orally or parenterally as injection or the like. Its dose may be from 0.01 to 900 mg/60 kg/day, preferably from 0.1 to 200 mg/60 kg/day, per an adult. The dose of an anticholinergic agent may be from 0.01 to 500 mg/60 kg/day, preferably from 0.2 to 100 mg/60 kg/day, per an adult.

Embodiments of the present invention are described below with reference to the following Examples, which, however do not restrict the scope of the invention.

### Best Modes for Carrying Out the Invention

### Example 1: Tablets (Compound (Ia))

Tablets having the following compositions were prepared according to an ordinary method.

Compound (Ia) (250 g), mannitol (1598.5 g), sodium starch glycolate (100 g), light silicic anhydride (10 g), magnesium stearate (40g) and yellow iron oxide (1.5g) were mixed according to an ordinary method. The resulting mixture was compressed using a tableting machine with 8 mm diameter punch and die (Purepress Correct-12, Kikusui Seisakusho) to prepare tablets (containing 25 mg of the active ingredient per tablet).

| Formulation | |
|---|---|
| Compound (Ia) | 25 mg |
| Mannitol | 159.85 mg |
| Sodium starch glycolate | 10 mg |
| Light silicic anhydride | 1 mg |
| Magnesium stearate | 4 mg |
| Yellow iron oxide | 0.15 mg |
| | 200 mg |

### Example 2: Capsules (Compound (Ia))

Capsules having the following compositions were prepared according to an ordinary method.

Compound (Ia) (500 g), lactose (300 g), light silicic anhydride (100 g) and sodium lauryl sulfate (100 g) were mixed according to an ordinary manner. The resulting mixture was encapsulated in hard capsules No. 1 (content: 100 mg/capsule) using a capsule filler (LZ-64, Zanasi) to prepare capsules (containing 50 mg of the active ingredient per capsule).

**Formulation**

| | |
|---|---|
| Compound (Ia) | 50 mg |
| Lactose | 30 mg |
| Light silicic anhydride | 10 mg |
| Sodium lauryl sulfate | 10 mg |
| | 100 mg |

### Example 3: Injection (Compound (Ia))

An injection having the following compositions is prepared according to an ordinary method.

Compound (Ia) (1 g) and D-mannitol (5 g) are added to and mixed with distilled water for injection, and an aqueous hydrochloric acid solution and an aqueous sodium hydroxide solution are added thereto to control pH of the resulting solution to 6. Then, distilled water for injection is added thereto to make the content 1000 ml in total. Under a germ-free condition, 2 ml of the resulting mixture is put into each glass vial, and an injection is thus obtained (containing 2 mg of the active ingredient per vial).

| Formulation | |
|---|---|
| Compound (Ia) | 2 mg |
| D-mannitol | 10 mg |
| Aqueous hydrochloric acid solution | proper quantity |
| Aqueous sodium hydroxide solution | proper quantity |
| Distilled water for injection | proper quantity |
| | 2.00 ml |

### Example 4: Tablets (tolterodine tartrate)

Tablets having the following composition are prepared according to an ordinary method.

Tolterodine tartrate (4 g), lactose (300 g) and potato starch (68 g) are mixed, and a 10% aqueous hydroxypropyl cellulose solution (200 g) is added to the mixture. The resulting mixture is kneaded according to an ordinary manner, then granulated and dried to give granules to be tabletted. 8.0 g of magnesium stearate is added thereto, and using a tabletting machine with 8 mm diameter punch and die (RT-15 Model, Kikusui Seisakusho), the resulting mixture is tabletted to give tablets (containing 0.2 mg of the active ingredient per tablet) .

| Formulation | |
|---|---|
| Tolterodine tartrate | 0.2 mg |
| Lactose | 150 mg |
| Potato starch | 34 mg |
| Hydroxypropyl cellulose | 10 mg |
| Magnesium stearate | 4 mg |
| | 200 mg |

### Example 5: Tablets (single preparation of Compound (Ia) and tolterodine tartrate)

Tablets having the following composition are prepared according to an ordinary method.

Compound (Ia) (40 g), tolterodine tartrate (4 g), lactose (286 g) and potato starch (56 g) are mixed, and a 10% aqueous hydroxypropyl cellulose solution (120 g) is added to the mixture. The resultingmixture is kneaded according to an ordinary manner, then granulated and dried to give granules to be tabletted. 2 g of magnesium stearate is added thereto, and using a tabletting machine with 8 mm diameter punch and die (RT-15 Model, Kikusui Seisakusho), the resulting mixture is tabletted to give tablets (containing 20 mg of compound (Ia) and 2 mg of tolterodine tartrate per tablet).

| Formulation | |
|---|---|
| Compound (Ia) | 20 mg |
| Tolterodine tartrate | 2 mg |
| Lactose | 143 mg |
| Potato starch | 28 mg |
| Hydroxypropyl cellulose | 6 mg |
| Magnesium stearate | 1 mg |
| | 200 mg |

### Example 6: Tablets (oxybutynin hydrochloride)

Tablets having the following composition are prepared according to an ordinary method.

Oxybutyninhydrochloride (4g), lactose (300 g) and potato starch (68 g) are mixed, and a 10% aqueous hydroxypropyl cellulose solution (200 g) is added to the mixture. The resulting mixture is kneaded according to an ordinary manner, then granulated and dried to give granules to be tabletted. 8 g of magnesium stearate is added thereto, and using a tablet ting machine with 8 mm diameter punch and die (RT-15 Model, Kikusui Seisakusho), the resulting mixture is tabletted to give tablets (containing 2 mg of the active ingredient per tablet).

| Formulation | |
|---|---|
| Oxybutynin hydrochloride | 2 mg |
| Lactose | 150 mg |
| Potato starch | 34 mg |
| Hydroxypropyl cellulose | 10 mg |
| Magnesium stearate | 4 mg |
| | 200 mg |

### Example 7: Tablets (single preparation of Compound (Ia) and oxybutynin hydrochloride)

Tablets having the following composition are prepared according to an ordinary method.

Compound (Ia) (40 g), oxybutynin hydrochloride (4 g), lactose (286 g) and potato starch (56 g) are mixed, and a 10% aqueous hydroxypropyl cellulose solution (120 g) is added to the mixture. The resulting mixture is kneaded according to an ordinary manner, then granulated and dried to give granules to be tabletted. 2 g of magnesium stearate is added thereto, and using a tabletting machine with 8 mm diameter punch and die (RT-15 Model, Kikusui Seisakusho), the resulting mixture was tabletted to give tablets (containing 20.0 mg of compound (Ia) and 2 mg of oxybutynin hydrochloride per tablet).

| Formulation | |
|---|---|
| Compound (Ia) | 20 mg |
| Oxybutynin hydrochloride | 2 mg |
| Lactose | 143 mg |
| Potato starch | 28 mg |
| Hydroxypropyl cellulose | 6 mg |
| Magnesium stearate | 1 mg |
| | 200 mg |

### Industrial Applicability

The present invention provides a pharmaceutical composition which comprises
3,3,3-trifluoro-2-hydroxy-2-methyl-N-(5,5,10-trioxo-4,10-dihydrothieno[3,2-c][1]benzothiepin-9-yl)propanamide or a pharmaceutically acceptable salt thereof, and an anticholinergic agent.

## Claims

1. A pharmaceutical composition which comprises (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 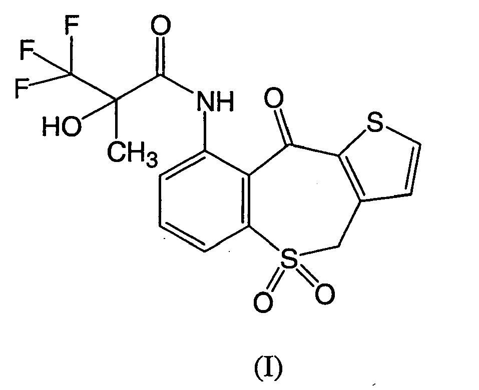 or a pharmaceutically acceptable salt thereof, and (b) an anticholinergic agent.

2. The pharmaceutical composition according to Claim 1, wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

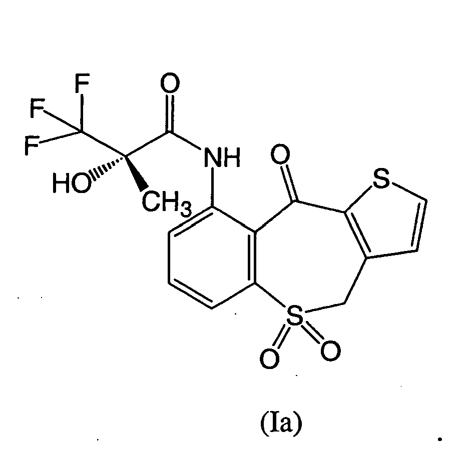

3. The pharmaceutical composition according to Claim 1 or 2, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

4. A therapeutic agent for overactive bladder, which comprises, as active ingredients, (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 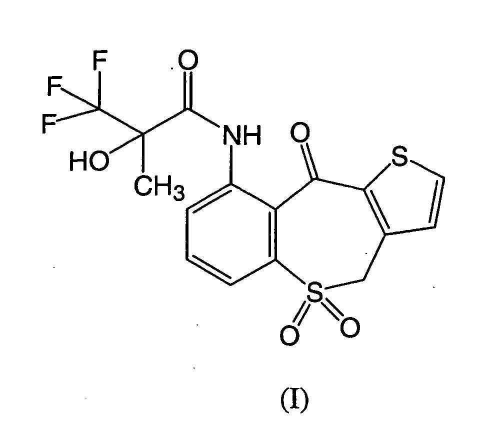 or a pharmaceutically acceptable salt thereof, and (b) an anticholinergic agent, which may be administered together or separately at an interval.

5. The therapeutic agent for overactive bladder according to Claim 4, wherein
3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by formula (Ia):

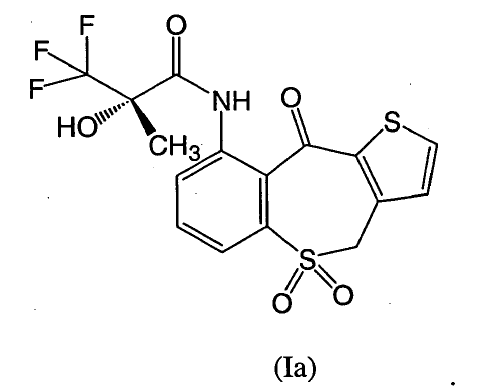

6. The therapeutic agent for overactive bladder according to Claim 4 or 5, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

7. A kit which comprises (a) a first component containing 3,3,3-trifluoro-2-hydroxy-2-methyl-N-(5,5,10-trioxo-4,10-dihydrothieno[3,2-c][1]benzothiepin-9-yl)propanamide represented by Formula (I): 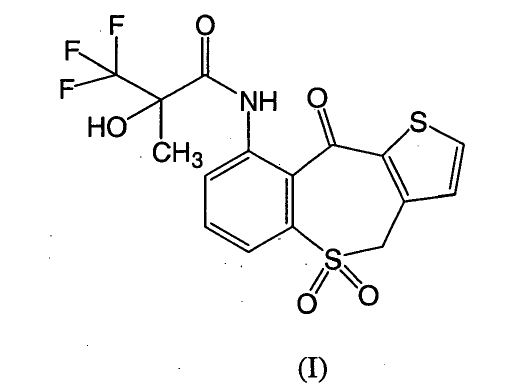 or a pharmaceutically acceptable salt thereof, and (b) a second component containing an anticholinergic agent.

8. The kit according to Claim 7, wherein
3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][l]benzothiepin-9-yl)propanamide represented by Formula (Ia):

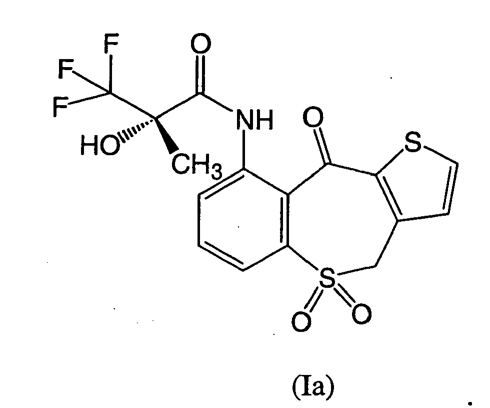

9. The kit according to Claim 7 or 8, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

10. A kit for treating overactive bladder, which comprises (a) a first component containing
3,3,3-trifluoro-2-hydroxy-2-methyl-N-(5,5,10-trioxo-4,10-dihydrothieno[3,2-c][1]benzothiepin-9-yl)propanamide represented by Formula (I): 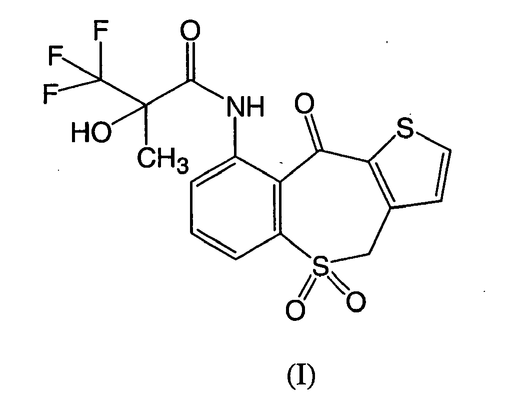 or a pharmaceutically acceptable salt thereof, and (b) a second component containing an anticholinergic agent.

11. The kit for treating overactive bladder according to Claim 10, wherein
3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

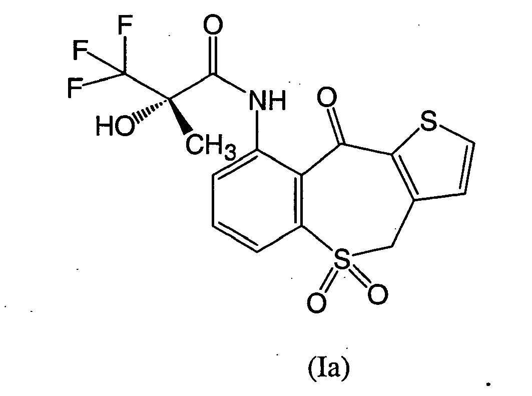

12. The kit for treating overactive bladder according to Claim 10 or 11, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

13. 3,3,3-Trifluoro-2-hydroxy-2-methyl-N-(5,5,10-trioxo-4,10-dihydrothieno[3,2-c][1]benzothiepin-9-yl)propanamide represented by Formula (I): 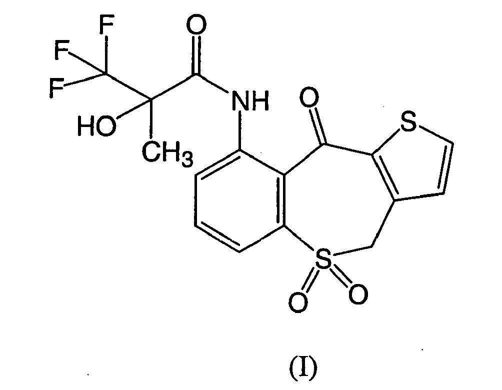 or a pharmaceutically acceptable salt thereof, which may be administered together or separately at an interval with an anticholinergic agent.

14. 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to Claim 13, wherein
3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

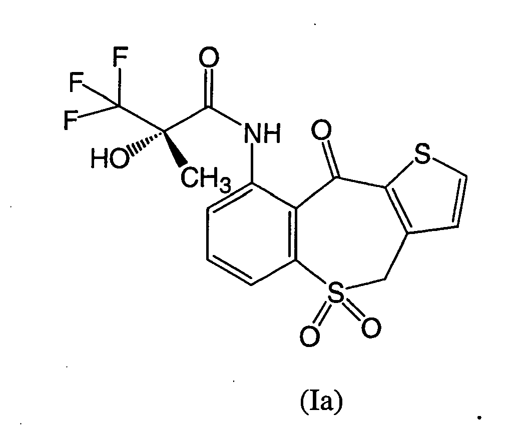

15. 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to Claim 13 or 14, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

16. A pharmaceutical composition which comprises, as an active ingredient,
3,3,3-trifluoro-2-hydroxy-2-methyl-N-(5,5,10-trioxo-4,10-dihydrothieno[3,2-c][1]benzothiepin-9-yl)propanamide represented by Formula (I): 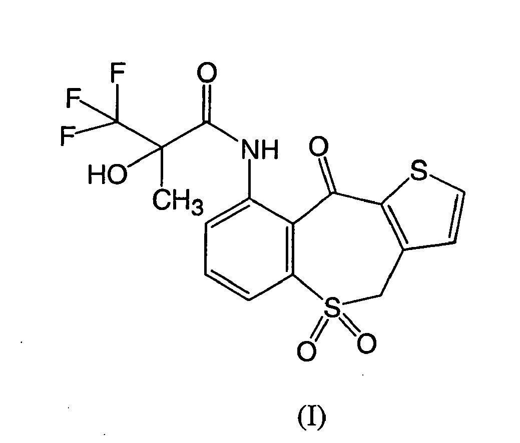 or a pharmaceutically acceptable salt thereof, which may be administered together or separately at an interval with an anticholinergic agent.

17. The pharmaceutical composition according to Claim 16, wherein
3,3,3-trifluoro-2-hydroxy-2-methyl-N-(5,5,10-trioxo-4,10-, dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

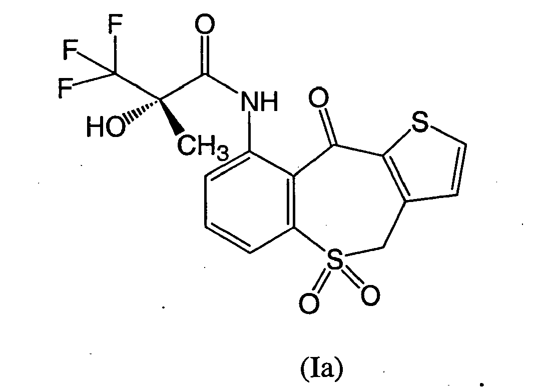

18. The pharmaceutical composition according to Claim 16 or 17, wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.

19. A method for treating overactive bladder, which comprises administering (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 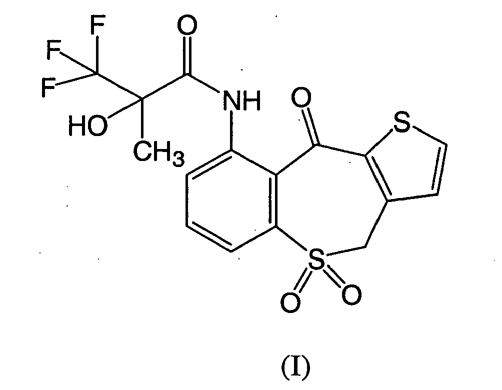 or a pharmaceutically acceptable salt thereof, and (b) an anticholinergic agent, which may be administered together or separately at an interval.

20. The method for treating overactive bladder according to Claim 19, wherein 3,3,3-trifluoro-2-hydroxy-2-methyl-N-(5,5,10-trioxo-4,10-dibydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

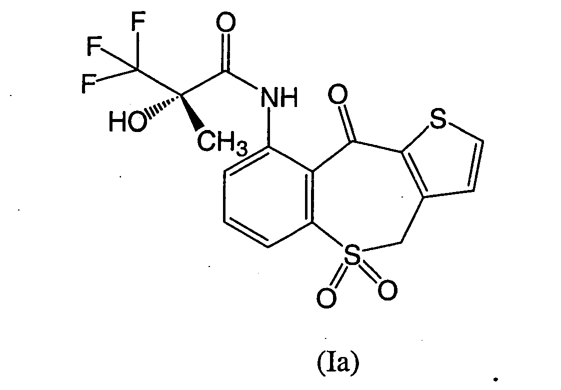

21. The method for treating overactive bladder according to Claim 19 or 20 , wherein the anticholinergic agent is/are selected from any one of or more than one of the following: oxybutynin, propiverine, tolterodine, darifenacin, temiverine, trospium chloride, tiotropium, oxytropium, ipratropium, flutropium, atropine, scopolamine, solifenacin and KRP-197, and pharmaceutically acceptable salts thereof.
